# EUROPEAN PATENT APPLICATION

(11) **EP 3 590 444 A2**
(43) Date of publication of application: **08.01.2020**
(21) Application number: 19184366.3
(22) Date of filing: 04.07.2019
(51) Int. Cl.: A61B 17/128

(54) **END EFFECTOR ASSEMBLIES, DRIVE SLEEVES, AND SURGICAL CLIP APPLIERS INCORPORATING THE SAME**

(30) Priority: 05.07.2018 US 201862694236 P; 29.03.2019 US 201916369083
(71) Applicant: Covidien LP, Mansfield, MA 02048 (US)
(72) Inventor: DININO, Matthew A., Newington, CT Connecticut 06111 (US); CRESTON, Brian J., Madison, CT Connecticut 06443 (US); BARIL, Jacob C., Norwalk, CT Connecticut 06851 (US); MALAVENDA, Matthew, West Haven, CT Connecticut 06516 (US); ZAMMATARO, Thomas A., Hamden, CT Connecticut 06517 (US)
(74) Representative: Maschio, Antonio

(57) **Abstract**

A surgical clip applier includes an outer shaft, an end effector assembly, an inner drive sleeve disposed, and a pin. The end effector assembly includes first and second jaw components. The inner drive sleeve is disposed within the outer shaft and about the jaw components. The inner drive sleeve is movable from a proximal position to a distal position to cam about outer surfaces of the jaw components to move first and second jaws from a spaced-apart position to an approximated position to apply a surgical clip. The pin is disposed between the jaw components. Movement of the inner drive sleeve from the distal position back to the proximal position cams the pin about inner surfaces of the jaw components to move the first and second jaws from the approximated position back to the spaced-apart position.

## Description

### CROSS-REFERENCE TO RELATED APPLICATION

This application claims the benefit of and priority to U.S. Provisional Patent Application No. 62/694,236 filed July 5, 2018, the entire disclosure of which is incorporated by reference herein.

### BACKGROUND

### Technical Field

The present disclosure relates to surgical clip appliers. More particularly, the present disclosure relates to end effector assemblies, drive sleeves, and surgical clip appliers including the same.

### Description of Related Art

Surgical clip appliers are known in the art and are used for a number of distinct and useful surgical procedures. In the case of a laparoscopic surgical procedure, access to the interior of an abdomen is achieved through narrow tubes or cannulas inserted through a small entrance incision in the skin. Minimally invasive procedures performed elsewhere in the body are often generally referred to as endoscopic procedures.

Endoscopic surgical clip appliers having various sizes (e.g., diameters), that are configured to apply a variety of diverse surgical clips, are also known in the art, and are capable of applying a single or multiple surgical clips during an entry to the body cavity. Such surgical clips are typically fabricated from a biocompatible material and are usually compressed over tissue. Once applied to tissue, the compressed surgical clip terminates the flow of fluid therethrough.

### SUMMARY

As detailed herein and shown in the drawing figures, as is traditional when referring to relative positioning on a surgical instrument, the term "proximal" refers to the end of the apparatus or component thereof which is closer to the user and the term "distal" refers to the end of the apparatus or component thereof which is further away from the user. Further, to the extent consistent, any or all of the aspects and features detailed herein may be used in conjunction with any or all of the other aspects and features detailed herein.

Provided in accordance with aspects of the present disclosure is a surgical clip applier including an outer shaft, an end effector assembly supported within the outer shaft and extending distally from the outer shaft, an inner drive sleeve, and a pin. The end effector assembly includes first and second jaw components coupled to one another at proximal portions of the first and second jaw components. The first and second jaw components include respective first and second arms extending distally from the respective proximal portions thereof and respective first and second jaws disposed at free ends of the first and second arms, respectively.

The inner drive sleeve is disposed within the outer shaft and about the first and second jaw components. The inner drive sleeve is movable within the outer shaft and relative to the first and second jaw components from a proximal position to a distal position to cam about outer surfaces of the first and second jaw components to move the first and second jaws from a spaced-apart position to an approximated position to apply a surgical clip about tissue disposed between the first and second jaws.

The pin is supported by the inner drive sleeve and disposed between the first and second arms of the first and second jaw components. Movement of the inner drive sleeve from the distal position back to the proximal position cams the pin about inner surfaces of the first and second jaw components to move the first and second jaws from the approximated position back to the spaced-apart position.

In an aspect of the present disclosure, the pin defines a wedge-shaped configuration increasing in width in a proximal-to-distal direction to facilitate camming about the inner surfaces of the first and second jaw components to move the first and second jaws from the approximated position back to the spaced-apart position.

In another aspect of the present disclosure, the pin defines a tear-drop-shaped configuration.

In another aspect of the present disclosure, the arm of at least one of the first or second jaw components defines a cut-out within the inner surface thereof. The at least one cut-out is configured to at least partially receive the pin therein in the approximated position of the first and second jaws.

In still another aspect of the present disclosure, the arm of each of the first and second jaw components defines a cut-out within the inner surface thereof. The cut-outs are configured to at least partially receive the pin therein in the approximated position of the first and second jaws.

In yet another aspect of the present disclosure, the first and second jaw components are pivotably coupled to one another and the outer shaft at the proximal portions thereof via a pivot pin. The first and second jaw components are pivotable about the pivot pin to move the first and second jaws between the spaced-apart and approximated positions.

In still yet another aspect of the present disclosure, the pivot pin extends through opposed slots defined within the inner drive sleeve to enable slidable movement of the inner drive sleeve about the pivot pin.

Another surgical clip applier provided in accordance with aspects of the present disclosure includes an outer shaft, an end effector assembly supported within the outer shaft and extending distally from the outer shaft, an inner drive sleeve, and a wedge-shaped pin. The end effector assembly includes first and second jaw components movable between a spaced-apart position and an approximated position to apply a surgical clip about tissue disposed therebetween.

The inner drive sleeve is disposed within the outer shaft and about the first and second jaw components. The inner drive sleeve is movable within the outer shaft and relative to the first and second jaw components from a proximal position to a distal position to cam about outer surfaces of the first and second jaw components to move the first and second jaw components from the spaced-apart position to the approximated position.

The wedge-shaped pin increases in width in a proximal-to-distal direction, is supported by the inner drive sleeve, and is disposed between the first and second jaw components. Movement of the inner drive sleeve from the distal position back to the proximal position cams the pin about inner surfaces of the first and second jaw components to move the first and second jaws components from the approximated position back to the spaced-apart position.

In an aspect of the present disclosure, the pin defines a tear-drop-shaped configuration.

In another aspect of the present disclosure, at least one of the first or second jaw components defines a cut-out within the inner surface thereof. The at least one cut-out is configured to at least partially receive the pin therein in the approximated position of the first and second jaw components.

In still another aspect of the present disclosure, each of the first and second jaw components defines a cut-out within the inner surface thereof. The cut-outs are configured to at least partially receive the pin therein in the approximated position of the first and second jaw components.

In yet another aspect of the present disclosure, the first and second jaw components are pivotably coupled to one another and the outer shaft via a pivot pin. The first and second jaw components are pivotable about the pivot pin between the spaced-apart and approximated positions.

In still yet another aspect of the present disclosure, the pivot pin extends through opposed slots defined within the inner drive sleeve to enable slidable movement of the inner drive sleeve about the pivot pin.

Another surgical clip applier provided in accordance with aspects of the present disclosure includes an outer shaft, an end effector assembly supported within the outer shaft and extending distally from the outer shaft, and an inner drive sleeve. The end effector assembly includes first and second jaw components movable between a spaced-apart position and an approximated position to apply a surgical clip about tissue disposed therebetween. At least one of the first or second jaw components defines a cut-out within an inner surface thereof.

The inner drive sleeve is disposed within the outer shaft and about the first and second jaw components. The inner drive sleeve supports a pin disposed between the first and second jaw components. The inner drive sleeve is movable within the outer shaft and relative to the first and second jaw components from a proximal position to a distal position to cam about outer surfaces of the first and second jaw components to move the first and second jaw components from the spaced-apart position to the approximated position.

The pin is at least partially received within the at least one cut-out in the approximated position of the first and second jaw components. Movement of the inner drive sleeve from the distal position back to the proximal position cams the pin about inner surfaces of the first and second jaw components to move the first and second jaw components from the approximated position back to the spaced-apart position.

In an aspect of the present disclosure, the pin defines a wedge-shaped configuration increasing in width in a proximal-to-distal direction to facilitate camming about the inner surfaces of the first and second jaw components to move the first and second jaw components from the approximated position back to the spaced-apart position.

In another aspect of the present disclosure, the pin defines a tear-drop-shaped configuration.

In still another aspect of the present disclosure, each of the first and second jaw components defines a cut-out within the inner surface thereof. The cut-outs are configured to at least partially receive the pin therein in the approximated position of the first and second jaw components.

In yet another aspect of the present disclosure, the first and second jaw components are pivotably coupled to one another and the outer shaft via a pivot pin. The first and second jaw components are pivotable about the pivot pin between the spaced-apart and approximated positions.

In still yet another aspect of the present disclosure, the pivot pin extends through opposed slots defined within the inner drive sleeve to enable slidable movement of the inner drive sleeve about the pivot pin.

### BRIEF DESCRIPTION OF THE DRAWINGS

Aspects and features of the present disclosure are described in detail with reference to the drawing figures wherein like reference numerals identify similar or identical structural elements and:
FIG. 1 is a front, perspective view of a surgical clip applier provided in accordance with the present disclosure including a handle assembly having an elongated assembly engaged therewith;
FIG. 2 is front, perspective view of the surgical clip applier with the elongated assembly removed from the handle assembly;
FIG. 3A is an enlarged, side view of the handle assembly of the surgical clip applier with a portion of the housing thereof removed to illustrate the internal components and features therein, wherein the trigger is disposed in an un-actuated position;
FIG. 3B is an enlarged, side view of the handle assembly of the surgical clip applier with a portion of the housing thereof removed to illustrate the internal components and features therein, wherein the trigger is disposed in an actuated position;
FIG. 4 is a side view of the surgical clip applier with the portion of the housing of the handle assembly removed;
FIG. 5 is a side, perspective view, with portions shown transparent, of a distal portion of the elongated assembly;
FIG. 6 is a side view, with portions shown transparent, of the distal portion of the elongated assembly;
FIG. 7 is a longitudinal, cross-sectional view of the distal portion of the elongated assembly;
FIG. 8 is a perspective view of another end effector assembly configured for use with the elongated assembly;
FIG. 9 is an exploded, perspective view of the end effector assembly of FIG. 8;
FIG. 10 is a side, perspective view of a distal portion of an inner drive sleeve provided in accordance with the present disclosure shown operably coupled about the end effector assembly of FIG. 8;
FIG. 11 is a longitudinal, cross-sectional view of the distal portion of the inner drive sleeve of FIG. 10 shown operably coupled about the end effector assembly of FIG. 8;
FIG. 12 is a side view of the distal portion of the inner drive sleeve of FIG. 10 shown operably coupled about the end effector assembly of FIG. 8 and disposed in a proximal position relative thereto;
FIG. 13 is a side view of the distal portion of the inner drive sleeve of FIG. 10 shown operably coupled about the end effector assembly of FIG. 8 and disposed in a distal position relative thereto;
FIG. 14 is a side, perspective view of a distal portion of another inner drive sleeve provided in accordance with the present disclosure shown operably coupled about another end effector assembly provided in accordance with the present disclosure;
FIG. 15 is an exploded, perspective view of the inner drive sleeve and end effector assembly of FIG. 14;
FIG. 16 is a longitudinal, cross-sectional view of the inner drive sleeve and end effector assembly of FIG. 14 illustrating distal movement of the inner drive sleeve about the end effector assembly to move the jaws of the end effector assembly towards an approximated position; and
FIG. 17 is a longitudinal, cross-sectional view of the inner drive sleeve and end effector assembly of FIG. 14 illustrating proximal movement of the inner drive sleeve about the end effector assembly to return the jaws of the end effector assembly towards a spaced-apart position.

### DETAILED DESCRIPTION

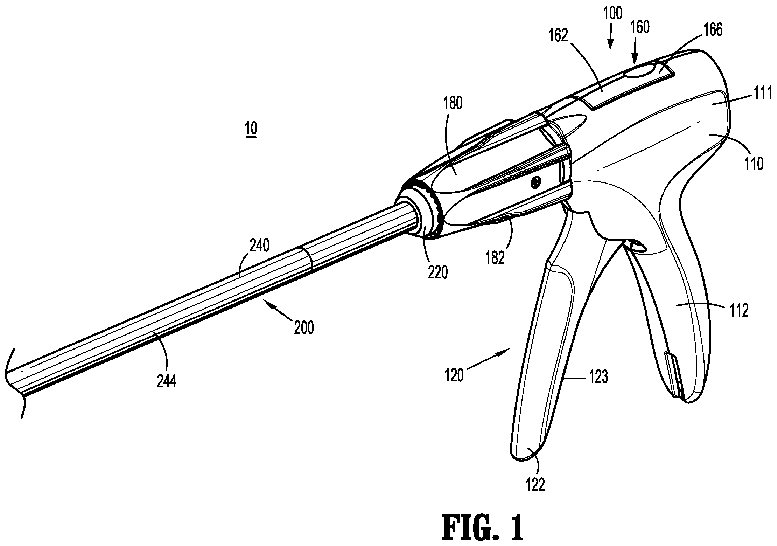

Turning to FIGS. 1-4, a surgical clip applier embodying the aspects and features of the present disclosure is shown generally identified by reference numeral 10. Surgical clip applier 10 generally includes a handle assembly 100 and an elongated assembly 200 selectively connectable to handle assembly 100. Handle assembly 100 is configured to operate elongated assembly 200 upon connection thereto, and may be configured as a sterilizable, reusable component such that handle assembly 100 may be repeatedly used with different and/or additional elongated assemblies 200 during the course of one or more surgical procedures. Elongated assembly 200 may be configured as single-use disposable component, limited-use disposable component, or reusable component, depending upon a particular purpose.

Handle assembly 100 generally includes a housing 110, an actuation mechanism 120 operably associated with housing 110, a latch assembly 160 operably associated with housing 110, and a rotating receiver assembly 180 operably coupled to a distal portion of housing 110. Housing 110 of handle assembly 100 supports and/or encloses the operating components of handle assembly 100 and defines a body portion 111 and a fixed handle portion 112 depending from body portion 111. Body portion 111 of housing 110 includes an internal pivot post 114 extending transversely within body portion 111 and a distal opening 118 through which a proximal end portion of elongated assembly 200 extends when elongated assembly 200 is engaged with handle assembly 100.

Actuation mechanism 120 is operably supported by housing 110 and includes a trigger 122, a drive bar 130, and a linkage assembly 140. Trigger 122 includes a grasping portion 123, an intermediate pivot portion 124, and a proximal extension 125. Grasping portion 123 of trigger 122 extends downwardly from body portion 111 of housing 110 in opposed relation relative to fixed handle portion 112 of housing 110. Grasping portion 123 is configured to facilitate grasping and manipulation of trigger 122. Intermediate pivot portion 124 of trigger 122 is at least partially disposed within housing 110 and defines a pivot aperture 126 that is configured to receive pivot post 114 of housing 110 so as to enable pivoting of trigger 122 about pivot post 114 and relative to housing 110, e.g., between an un-actuated position, wherein grasping portion 123 of trigger 122 is spaced-apart relative to fixed handle portion 112, and an actuated position, wherein grasping portion 123 of trigger 122 is approximated relative to fixed handle portion 112.

Proximal extension 125 of trigger 122 is disposed on an opposite side of intermediate pivot portion 124 and, thus, pivot post 114, as compared to grasping portion 123 of trigger 122. As such, pivoting of grasping portion 123 to rotate in one direction, e.g., proximally towards fixed handle portion 112, pivots proximal extension 125 to rotate in the opposite direction, e.g., distally.

Linkage assembly 140 includes a first linkage 142, a second linkage 144, and a third linkage 146. First linkage 142 is pivotably coupled to proximal extension 125 of trigger 122 towards a first end 143a of first linkage 142. Second and third linkages 144, 146, respectively, are each pivotably coupled to a second end 143b of first linkage 142 at respective first ends 145a, 147a of second and third linkages 144, 146. A second end 145b of second linkage 144 is pivotably coupled to drive bar 130, while a second end 147b of third linkage 146 is pivotably coupled to body portion 111 of housing 110. Thus, the pivot point between first linkage 142 and proximal extension 125 of trigger 122, the pivot point between first linkage 142 and second and third linkages 144, 146, respectively, and the pivot point between second linkage 144 and drive bar 130 are movable pivot points (e.g., movable relative to housing 110), while the pivot point between third linkage 146 and housing 110 is a fixed pivot point (e.g., fixed relative to housing 110).

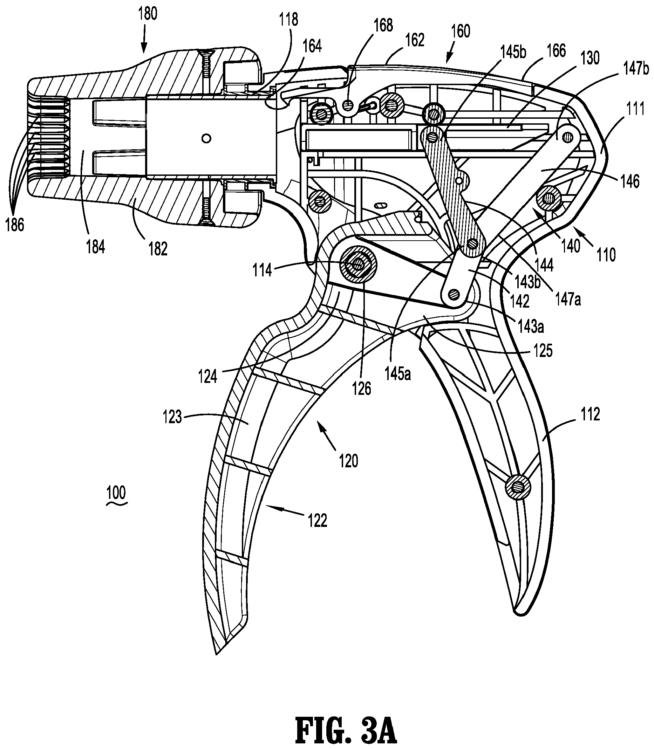

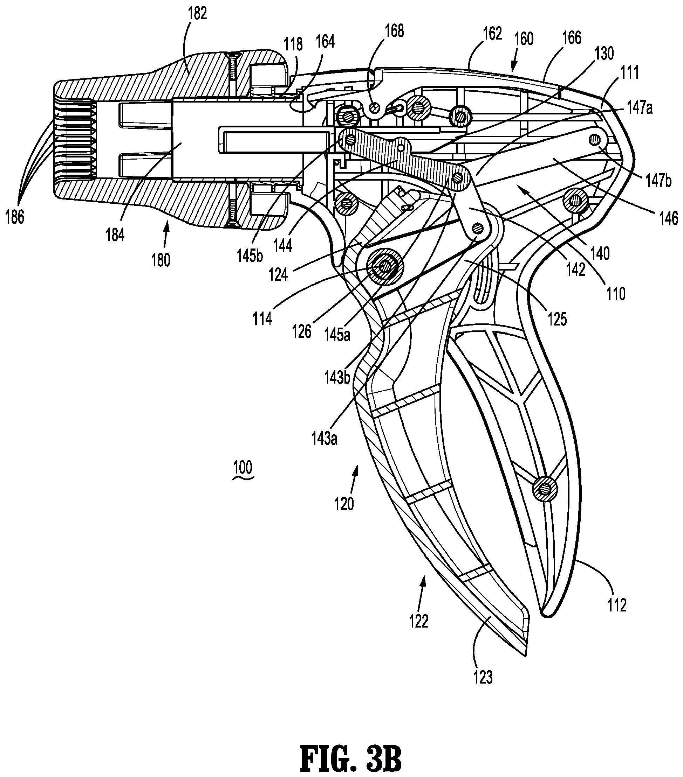

Upon actuation of trigger 122, e.g., proximal pivoting of grasping portion 123 of trigger 122, proximal extension 125 is moved in a counter-clockwise direction (from the orientation illustrated in FIG. 3), thereby urging first linkage 142 towards drive bar 130. This movement of first linkage 142 towards drive bar 130, in turn, urges first ends 145a, 147a of second and third linkages 144, 146, respectively, towards drive bar 130 to, in turn, urge second end 145b of second linkage 144 distally such that drive bar 130 is translated distally through body portion 111 of housing 110. A biasing spring (not shown) may be provided to bias trigger 122 towards an un-actuated position, thereby biasing drive bar 130 proximally.

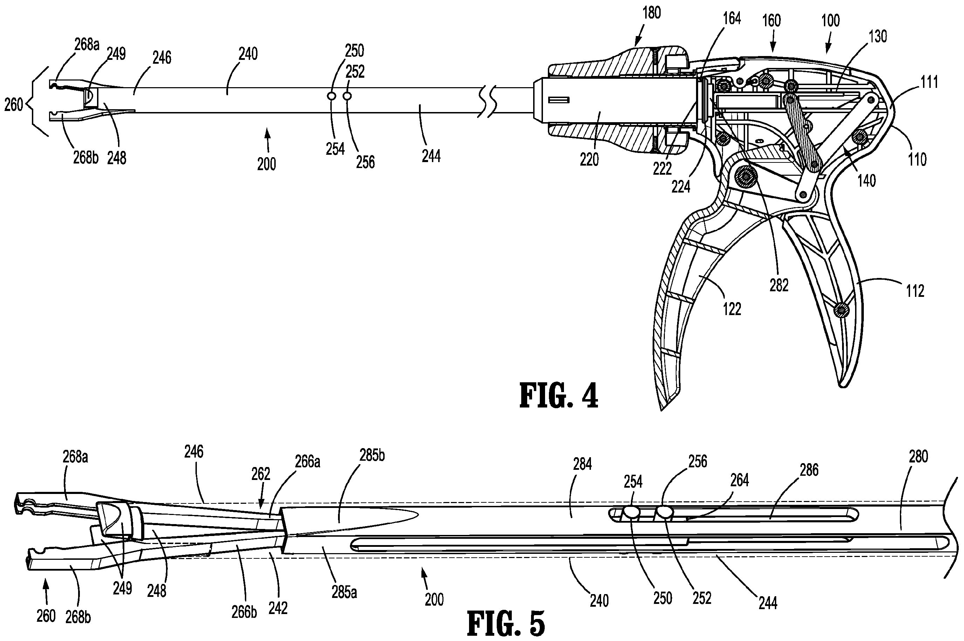

Drive bar 130 is slidably disposed within body portion 111 of housing 110 in longitudinal alignment with proximal portion 282 of inner drive sleeve 280 of elongated assembly 200 (see FIG. 4) when elongated assembly 200 is engaged with handle assembly 100 such that distal sliding of drive bar 130 through body portion 111 of housing urges drive bar 130 into contact with proximal portion 282 of inner drive sleeve 280 to thereby translate inner drive sleeve 280 distally, e.g., to apply, form or close a surgical clip supported at end effector assembly 260 of elongated assembly 200, as detailed below.

Latch assembly 160 is configured to facilitate releasable locking engagement of elongated assembly 200 with handle assembly 100. Latch assembly 160, more specifically, includes a pivoting lever arm 162 operably disposed on and extending into body portion 111 of housing 110. Lever arm 162 includes an engagement finger 164 disposed towards one end thereof and a manipulatable portion 166 disposed towards the other end thereof with a pivot portion 168 disposed therebetween. Thus, upon depression of manipulatable portion 166 into housing 110 from a locked position to an unlocked position, engagement finger 164 is withdrawn upwardly and, upon release of manipulatable portion 166 and return thereof to the locked position, engagement finger 164 is returned downwardly. A torsion spring (not shown) disposed about pivot portion 168, or other suitable biasing spring in any suitable position, may be provided to bias lever arm 162 towards the locked position, although other configurations are also contemplated.

Rotating receiver assembly 180 is configured to receive a proximal end portion of elongated assembly 200 and to enable selective rotation thereof relative to housing 110. Rotating receiver assembly 180 includes a rotation knob 182 rotatably coupled to body portion 111 of housing 110 and extending distally therefrom. Rotation knob 182 defines a lumen 184 extending therethrough in communication with distal opening 118 of body portion 111 of housing 110 to enable insertion of a proximal portion of elongated assembly 200 therethrough and into operable engagement within housing 110. Rotation knob 184 defines channels 186 disposed on an interior surface thereof and arranged annularly about lumen 184 to enable rotatable coupling of elongated assembly 200 therewith, as detailed below.

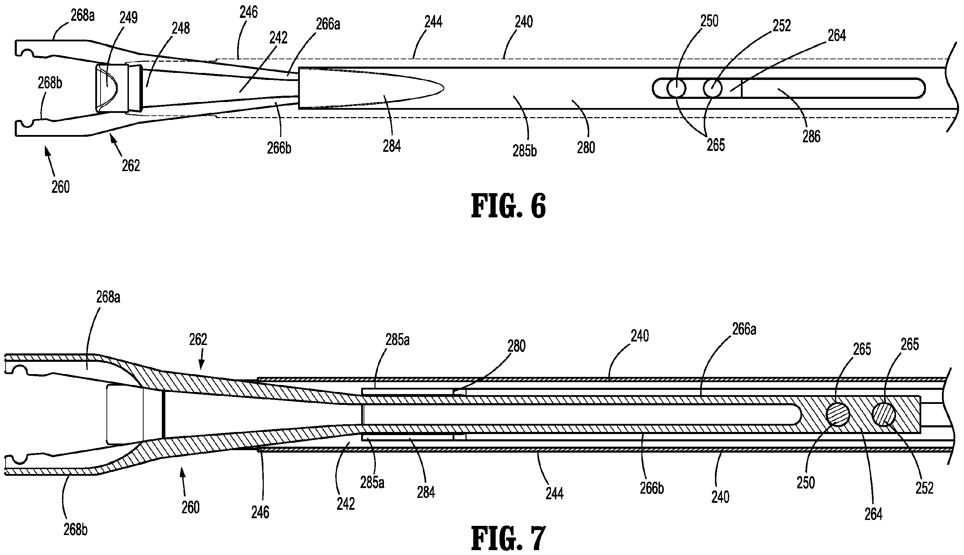

With additional reference to FIGS. 5-7, elongated assembly 200 generally includes a proximal hub 220, an elongated shaft 240 extending distally from proximal hub 220, an end effector assembly 260 disposed towards a distal end portion of elongated shaft 240, and an inner drive sleeve 280 slidably disposed through proximal hub 220 and elongated shaft 240 and configured for operable coupling between handle assembly 100 and end effector assembly 260 when elongated assembly 200 is engaged with handle assembly 100 to enable firing of a surgical clip (not shown) about tissue.

Proximal hub 220 is configured for insertion through lumen 184 of rotation knob 182 and into body portion 111 of housing 110. Proximal hub 220 defines an annular recess 222 towards the proximal end thereof and a chamfered proximal edge 224. Thus, upon insertion of proximal hub 220 through lumen 184 of rotation knob 182 and into body portion 111 of housing 110, chamfered proximal edge 224 cams engagement finger 164 of latch assembly 160 over the outer surface of proximal hub 220 until engagement finger 164 is disposed in alignment with annular recess 222, wherein engagement finger 164 falls into engagement within annular recess 222 to engage proximal hub 220 and, thus, elongated assembly 200, with handle assembly 100. As can be appreciated, in order to disengage and remove elongated assembly 200 from handle assembly 100, manipulatable portion 166 of latch assembly 160 is depressed into housing 110 to withdraw engagement finger 164 from annular recess 222 and enable elongated assembly 200 to be pulled distally and removed from handle assembly 100. Proximal hub 220 may further include a lock tab 226 extending along a portion of the length thereof and configured for receipt within one of the channels 186 defined within rotation knob 182 to rotationally fix elongated assembly 20 relative to rotation knob 182 upon insertion therein.

Elongated shaft 240 extends distally from proximal hub 220 and defines a longitudinal lumen 242 extending therethrough. Elongated shaft 240 further includes a body 244 and a bifurcated distal portion 246 including a pair of radially-opposed flanges 248 extending distally from body 244. Opposed flanges 248 define tissue stops 249 configured to inhibit passage of tissue into the space defined therebetween, as detailed below.

End effector assembly 260 of elongated assembly 200 is formed as a monolithic component of a single piece of material, e.g., via stamping or other suitable manufacturing process, and includes a jaws component 262 having a proximal base 264, a pair of spaced-apart arms 266a, 266b extending distally from proximal base 264, and a jaw 268a, 268b disposed at the free distal end of each arm 266a, 266b, respectively.

Proximal base 264 of jaws component 262 defines pair of apertures 265 extending transversely therethrough and in longitudinal alignment with one another, although greater or fewer apertures or otherwise arranged apertures are also contemplated. Apertures 265 are configured for receipt of pins 250, 252 which extend transversely through elongated shaft 240 and at least partially into opposed pairs of apertures 254, 256, respectively, defined transversely through elongated shaft 240. The portions of pins 250, 252 extending into or through apertures 254, 256 may be welded to elongated shaft 240 or otherwise engaged thereto to fix pins 250, 252 and, thus, proximal base 264 of jaws component 262 relative to elongated shaft 240.

Spaced-apart arms 266a, 266b of jaws component 262 extend distally from proximal base 264 to jaws 268a, 268b, respectively, and are resiliently flexible from an at-rest position, wherein spaced-apart arms 266a, 266b are angled apart from one another to define an increasing distance therebetween in the proximal-to-distal direction, to a flexed position, wherein spaced-apart arms 266a, 266b are closer to one another and disposed in a more-parallel orientation or angled towards one another. Spaced-apart arms 266a, 266b are oriented 90 degrees offset from flanges 248 of elongated shaft 240 to enable the portions of spaced-apart arms 266a, 266b disposed between flanges 248 to extend radially outwardly beyond the radial dimension of elongated shaft 240 in the at-rest position thereof without interference from flanges 248. This configuration also positions tissue stops 249 on the lateral sides of spaced-apart arms 266a, 266 to inhibit tissue ingress into the space defined between spaced-apart arms 266a, 266b.

Jaws 268a, 268b, as noted above, are disposed at the free distal ends of spaced-apart arms 266a, 266b, respectively. Jaws 268a, 268b may define transverse notches 270, longitudinal slots 272, and/or other suitable features to facilitate retention of legs of a surgical clip (not shown) therein. Jaws 268a, 268b are moved from a spaced-apart position to an approximated position upon movement of spaced-apart arms 266a, 266b from the at-rest position to the flexed position to thereby form a surgical clip held between jaws 268a, 268b about tissue disposed between jaws 268a, 268b. End effector assembly 260, in embodiments, may be configured to form surgical clips similar to those shown and described in U.S. Patent No. 4,834,096, the entire contents of which is hereby incorporated herein by reference.

Inner drive sleeve 280 defines a proximal portion 282 (FIG. 4) and a distal portion 284. Proximal portion 282 of inner drive sleeve 280 is configured for positioning adjacent a distal end of drive bar 130 of handle assembly 100 when elongated assembly 200 is engaged with handle assembly 100 (see FIG. 4) such that distal translation of drive bar 130 through housing 110 (e.g., upon actuation of trigger 122), urges drive bar 130 into contact with inner drive sleeve 280 to translate inner drive sleeve 280 distally through elongated shaft 240 of elongated assembly 200.

Distal portion 284 of inner drive sleeve 280 is slidably disposed about at least a proximal portion of jaws component 262 of end effector assembly 260 and defines a rectangular transverse cross-sectional configuration having a pair of narrow sides 285a and a pair of wide sides 285b. Opposed longitudinally-extending slots 286 are defined through wide sides 285b of distal portion 284 of inner drive sleeve 280 in alignment with one another. Slots 286 enable passage of pins 250, 252 therethrough while still enabling sliding of distal portion 284 of inner drive sleeve 280 through elongated shaft 240 and about end effector assembly 260. Distal portion 284 of inner drive sleeve 280 is oriented such that spaced-apart arms 266a, 266b of jaws component 262 are disposed adjacent opposed narrow sides 285a of distal portion 284 with, in embodiments, the width of opposed narrow sides 285a generally approximating the width of spaced-apart arms 266a, 266b to inhibit relative lateral motion between spaced-apart arms 266a, 266b, thereby inhibiting splay between jaws 268a, 268b.

Wide sides 285b of distal portion 284 of inner drive sleeve 280 define heights greater than the minimum distance between spaced-apart arms 266a, 266b but less than the maximum distance between spaced-apart arms 266a, 266b such that distal sliding of distal portion 284 of inner drive sleeve 280 about jaws component 262, e.g., in response to actuation of trigger 122, cams narrow sides 285a about the exterior surfaces of spaced-apart arms 266a, 266b to urge spaced-apart arms 266a, 266b towards one another from the at-rest position towards the flexed position, thereby moving jaws 268a, 268b from the spaced-apart position towards the approximated position to form or close a surgical clip positioned therebetween about tissue disposed between jaws 268a, 268b. Upon release or return of trigger 122, inner drive sleeve 280 is returned proximally, allowing spaced-apart arms 266a, 266b to resiliently return towards the at-rest position, thereby returning jaws 268a, 268b towards the spaced-apart position to enable loading of a subsequent surgical clip for formation or closing about tissue. A biasing spring (not shown) associated with elongated assembly 200 may be provided to bias inner drive sleeve 280 proximally such that, upon release of trigger 122, inner drive sleeve 280 is returned proximally. Other suitable biasing configurations are also contemplated.

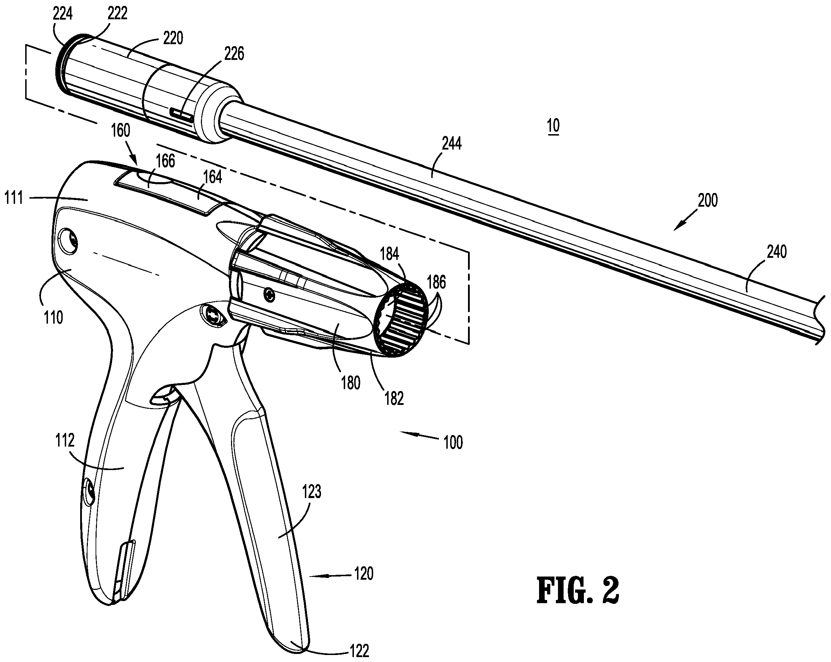

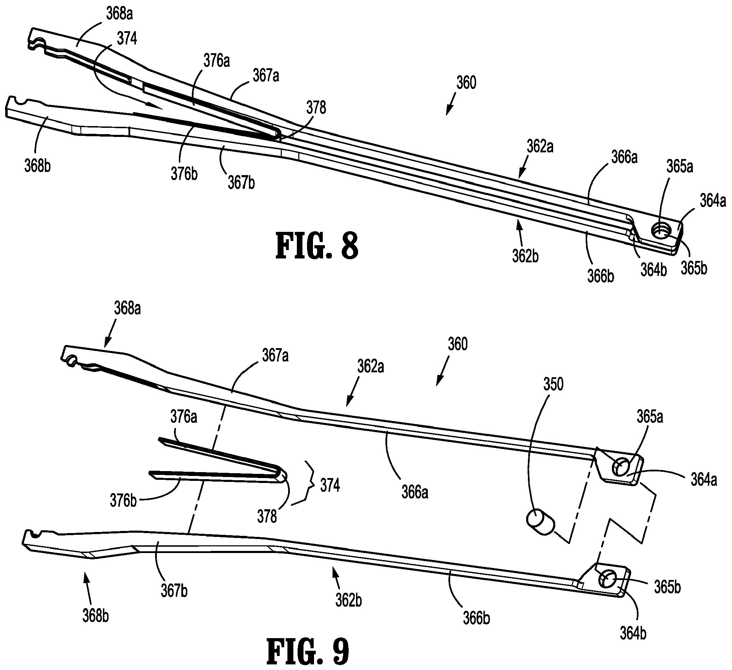

Turning to FIGS. 8 and 9, another embodiment of an end effector assembly provided in accordance with the present disclosure and configured for use with elongated assembly 200 (FIGS. 2 and 4-7) is shown generally identified by reference numeral 360. End effector assembly 360 includes first and second jaw components 362a, 362b, each including a proximal base 364a, 364b, an arm 366a, 366b extending distally from the respective proximal base 364a, 364b, and a jaw 368a, 368b disposed at the free distal end of the respective arm 366a, 366b. End effector assembly 360 further includes a leaf spring 374 including first and second legs 376a, 376b interconnected by a hinge 378. End effector assembly 360 may be similar to or include any of the features of end effector assembly 260 (FIGS. 5-7), except where specifically contradicted below.

Rather than providing a single, monolithic component as with jaws component 262 of end effector assembly 260 (see FIGS. 5-7), end effector assembly 360 includes separate first and second jaw components 362a, 362b. Proximal bases 364a, 364b of jaw components 362a 362b, respectively, are offset relative to respective arms 366a, 366b thereof such that jaw proximal bases 364a, 364b of jaw components 362a, 362b may be positioned in side-by-side relation relative to one another with arms 366a, 366b disposed in opposing alignment with one another. Proximal bases 364a, 364b further define aligned apertures 365a, 365b, respectively, extending transversely therethrough that are configured for receipt of a pin 350 to longitudinally fix and pivotably couple proximal bases 364a, 364b within the elongated shaft 240 (FIGS. 5-7), similarly as detailed above with respect to pins 250, 252, proximal base 264 of jaws component 262, and elongated shaft 240 (see FIGS. 5-7). Pin 350 also serves to pivotably couple proximal bases 364a, 364b with one another.

Arms 366a, 366b of end effector assembly 360 extend distally from respective proximal bases 364a, 364b. Arms 366a, 366b are identical to one another, with one arm 366a, 366b being inverted to face the other arm 366a, 366b. Arms 366a, 366b are substantially rigid in that arms 366a, 366b are not required to flex during proper operation of end effector assembly 360. Rather, arms 366a, 366b are pivotable relative to one another about pin 350 from a further-spaced position to a closer-together position. Each arm 366a, 366b includes a distal segment 367a, 367b, wherein jaws 368a, 368b extend distally from distal segments 367a, 367b of arms 366a, 366b, respectively.

Jaws 368a, 368b of end effector assembly 360 are similar to and may include any of the features of jaws 268a, 268b of end effector assembly 260, detailed above (see FIGS. 5-7), and are configured to move from a spaced-apart position towards an approximated position in response to movement of arms 366a, 366b from the further-spaced position towards the closer-together position to form or close a surgical clip about tissue. In accordance with the present disclosure, jaws 368a, 368b are structurally identical to one another.

Leaf spring 374 is configured for positioning between distal segments 367a, 367b of arms 366a, 366b with first and second legs 376a, 376b of leaf spring 374 abutting inwardly-facing surfaces of distal segments 367a, 367b of arms 366a, 366b, respectively, and extending distally from hinge 378. As such, leaf spring 374 biases arms 366a, 366b towards the further-spaced position and, thus, jaws 368a, 368b towards the spaced-apart position. First and second legs 376a, 376b of leaf spring 374 may be adhered or otherwise secured in engagement with the inwardly-facing surfaces of distal segments 367a, 367b of arms 366a, 366b, or may be retained therein via inner drive sleeve 280 (FIGS. 5-7) being disposed at least partially about distal segments 367a, 367b.

With additional reference to FIGS. 5-7, in use, distal sliding of distal portion 284 of inner drive sleeve 280 about jaw components 362a, 362b, e.g., in response to actuation of trigger 122 (FIG. 1), cams narrow sides 285a about the exterior surfaces of arms 366a, 366b to urge arms 366a, 366b to pivot about pin 350 towards one another from the further-spaced position towards the closer-together position, thereby moving jaws 368a, 368b from the spaced-apart position towards the approximated position to form or close a surgical clip positioned therebetween about tissue disposed between jaws 368a, 368b. The pivoting of arms 366a, 366b about pin 350 towards the closer-together position urges legs 376a, 376b of leaf spring 374 towards one another, against the bias of leaf spring 374. As such, upon release or return of trigger 122 (FIG. 1), inner drive sleeve 280 is returned proximally and jaws 368a, 368b and arms 366a, 366b are returned apart from one another towards the spaced-apart and further-spaced positions, respectively, under the bias of leaf spring 374 to enable loading of a subsequent surgical clip for formation or closure about tissue.

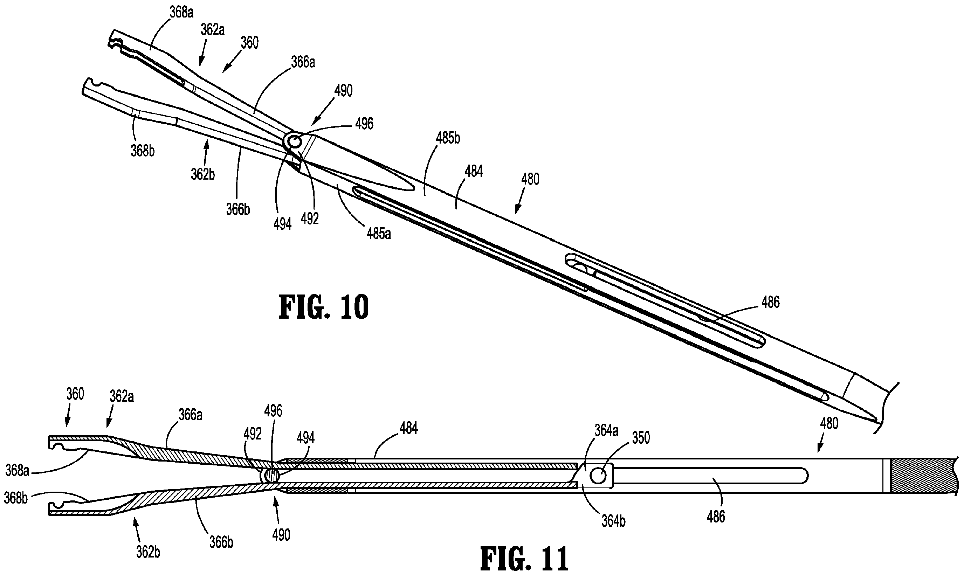

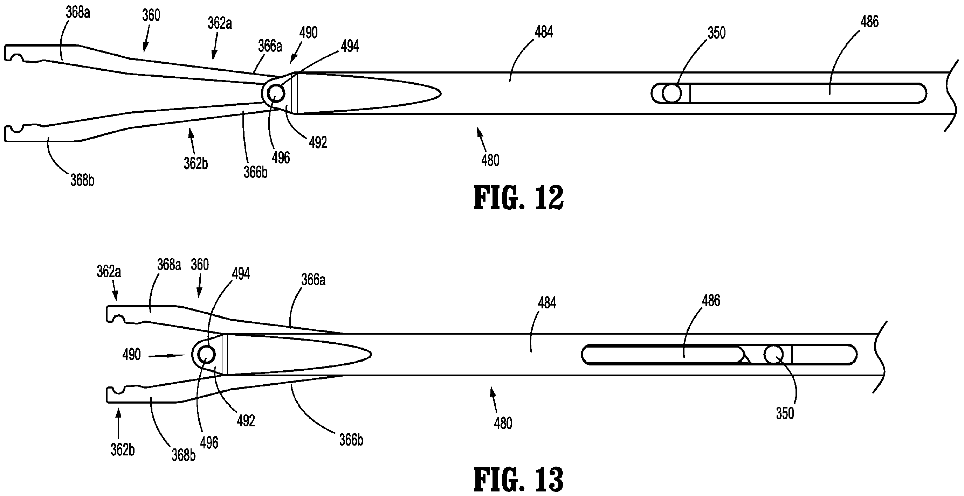

Turning to FIGS. 10-13, another embodiment of an inner drive sleeve provided in accordance with the present disclosure and configured for use with end effector assembly 260 of elongated assembly 200 (FIGS. 2 and 4-7) or end effector assembly 360 (as shown; see also FIGS. 8 and 9) is shown generally identified by reference numeral 480. Inner drive sleeve 480 includes a proximal portion (not shown) similar to proximal portion 282 of inner drive sleeve 280 (FIG. 4), and a distal portion 484.

Distal portion 484 of inner drive sleeve 480 is similar to distal portion 284 of inner drive sleeve 280 (FIGS. 5-7), slidably disposed about jaw components 362a, 362b of end effector assembly 360, and defines a rectangular transverse cross-sectional configuration. Opposed longitudinally-extending slots 486 are defined through wide sides 485b of distal portion 484 of inner drive sleeve 480 in alignment with one another. Slots 486 enable passage of pin 350 therethrough while still enabling sliding of distal portion 484 of inner drive sleeve 480 through elongated shaft 240 (FIGS. 5-7) and about end effector assembly 360.

Distal portion 484 of inner drive sleeve 480 further includes a clevis 490 extending distally from the distal ends of wide sides 485b of distal portion 484. Clevis 490, more specifically, includes a pair of clevis flanges 492 extending from wide sides 485b of distal portion 484 of inner drive sleeve 480 in spaced-apart relation relative to one another. Each clevis flange 492 defines an aperture 494 therethrough that is disposed in alignment with the aperture 494 of the other clevis flange 492. A pin 496 is received within apertures 494 and extends transversely between clevis flanges 492.

When end effector assembly 360 (or other suitable end effector assembly, e.g., end effector assembly 260 (FIGS. 5-7)) is assembled with inner drive sleeve 480, the pin 350 coupling proximal bases 364a, 364b of arms 366a, 366b extends through slots 486 of inner drive sleeve 480 to enable engagement of pin 350 with elongated shaft 240 (FIGS. 5-7) to pivotably couple arms 366a, 366b with one another and engage proximal bases 364a, 364b of arms 362a, 362b with elongated shaft 240 (FIGS. 5-7). Arms 366a, 366b extend distally from proximal bases 364a, 364b through inner drive sleeve 480, ultimately exiting inner drive sleeve 480 with arms 366a, 366b disposed on opposing sides of pin 496. Jaws 368a, 368b extend distally from arms 366a, 366b on either side of pin 496.

Pin 496 of clevis 490 of distal portion 484 of inner drive sleeve 480 defines a suitable diameter and is positioned, in the proximal position of inner drive sleeve 480, between arms 366a, 366b so as to function as a wedge maintaining arms 366a, 366b in the further-spaced position and, thus, jaws 368a, 368b in the spaced-apart position. As can be appreciated, as pin 496 is moved distally relative to the pivot point of jaws 368a, 368b, e.g., the location of pin 350, jaws 368a, 386b are permitted to pivot further towards one another whereas proximal movement of pin 496 relative to the pivot point of jaws 368a, 368b urges jaws 368a, 368b to pivot further apart from one another.

In use, distal sliding of distal portion 484 of inner drive sleeve 480 about arms 366a, 366b, e.g., in response to actuation of trigger 122 (FIG. 1), cams narrow sides 485a of inner drive sleeve 480 about the exterior surfaces of arms 366a, 366b to urge arms 366a, 366b to pivot about pin 350 towards one another from the further-spaced position towards the closer-together position, thereby moving jaws 368a, 368b from the spaced-apart position towards the approximated position to form or close a surgical clip positioned therebetween about tissue disposed between jaws 368a, 368b. This distal sliding of inner drive sleeve 480 relative to end effector assembly 360 moves pin 496 distally such that, as noted above, jaws 368a, 368b are permitted to pivot to the approximated position.

Upon release or return of trigger 122 (FIG 1), inner drive sleeve 480 is returned proximally and, thus, pin 496 is likewise returned proximally. As pin 496 is moved proximally towards the pivot point between jaws 368a, 368b, pin 496 eventually contacts the inwardly-facing surfaces of arms 366a, 366b, thereby functioning as a wedge to urge arms 366a, 366b to pivot apart from one another, thus urging jaws 368a, 368b to pivot towards the spaced-apart position. Thus, pin 496 serves to return jaws 368a, 368b to the spaced-apart position upon release or return of trigger 122 (FIG. 1) to enable loading of a subsequent surgical clip for formation or closure about tissue, without the need of a return biasing member such as leaf spring 374 (FIGS 8-9). Thus, in embodiments, end effector assembly 360, when used with inner drive sleeve 480, need not include leaf spring 374 (FIGS 8-9) or other return biasing member. As such, pin 496 returns jaws 368a, 368b to the spaced-apart position without imparting a return biasing force that is required to be overcome in order to approximate jaws 368a, 368b (such as the return biasing force provided by leaf spring 374 (FIGS. 8 and 9)). Thus, utilizing pin 496 to return jaws 368a, 368b to the spaced-apart position provides a decreased overall actuation force for approximating jaws 368a, 368b.

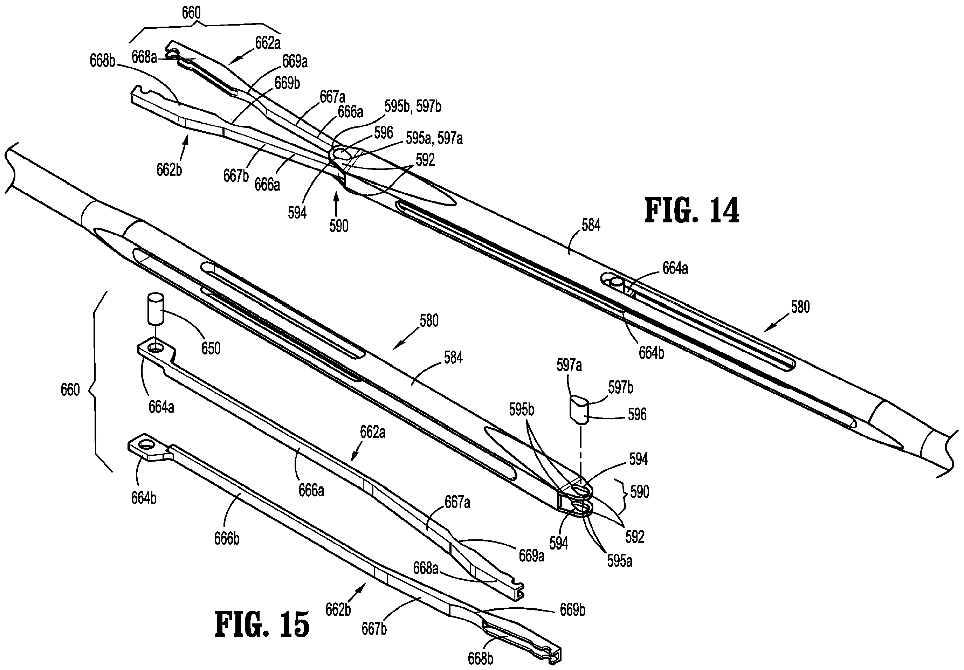

Turning to FIGS. 14-17, and initially to FIGS. 14 and 15, another embodiment of an inner drive sleeve provided in accordance with the present disclosure is shown generally identified by reference numeral 580 and configured for use with another end effector assembly provided in accordance with the present disclosure shown generally identified by reference numeral 660. Although inner drive sleeve 580 is shown and described herein for use with end effector assembly 660, it is contemplated that inner drive sleeve 580 may likewise be used with any other end effector assembly detailed herein (or any other suitable end effector assembly) and/or that end effector assembly 660 be used with any other inner drive sleeve detailed herein (or any other suitable inner drive sleeve).

Inner drive sleeve 580 is similar to inner drive sleeve 480 (FIGS. 10-13) and, thus, only differences therebetween are described in detail hereinbelow while similarities are summarily described or omitted entirely. Inner drive sleeve 580 includes a proximal portion (not shown) and a distal portion 584 having a clevis 590 extending distally therefrom. Clevis 590, more specifically, includes a pair of clevis flanges 592 extending from opposing sides of distal portion 584 of inner drive sleeve 580 in spaced-apart relation relative to one another. Clevis flanges 592 define apertures 594 therethrough in transverse alignment with one another. Apertures 594 define wedge-shaped configurations wherein the proximal ends of apertures 594 terminate at apexes 595a and wherein apertures 594 generally increase in width distally from the respective apexes 595a thereof towards the respective distal ends 595b thereof. In embodiments, distal ends 595b of apertures 594 define rounded configurations such that apertures 594 define tear-drop-shaped configurations. A pin 596 shaped complementary to apertures 594 and, thus, defining an apex 597a at the proximal end thereof and generally increasing in width distally from the apex 597a thereof to the distal end 579b thereof (and, in embodiments, defining a tear-drop-shaped configuration), is received within apertures 594 and extends transversely between clevis flanges 592. Pin 596 thus defines a wedge-shaped configuration increasing in width in a proximal-to-distal direction.

Continuing with reference to FIGS. 14 and 15, end effector assembly 660 is similar to end effector assembly 360 (FIGS. 8-10) and, thus, only differences therebetween are described in detail hereinbelow while similarities are summarily described or omitted entirely. End effector assembly 660 includes first and second jaw components 662a, 662b, each including a proximal base 664a, 664b, an arm 666a, 666b extending distally from the respective proximal base 664a, 664b, and a jaw 668a, 668b disposed at the free distal end of the respective arm 666a, 666b. Proximal bases 664a, 664b are pivotably coupled to one another via a pin 650.

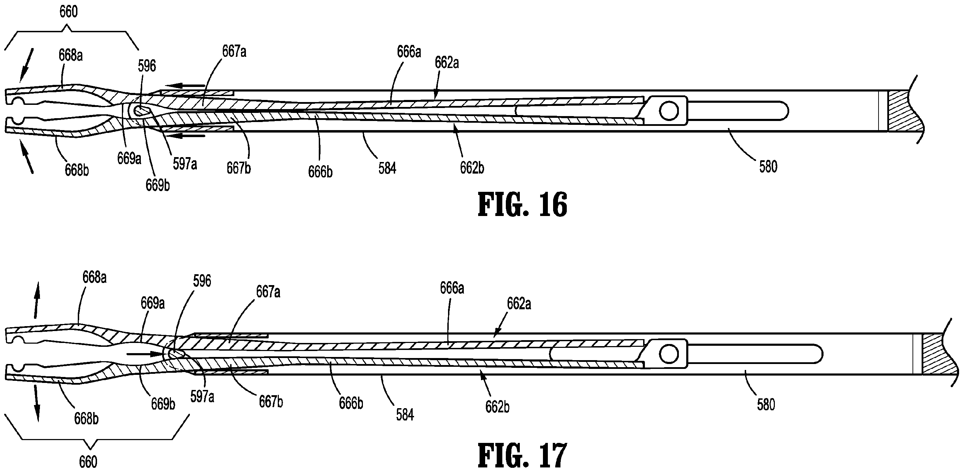

Arms 666a, 666b of end effector assembly 660 extend distally from respective proximal bases 664a, 664b. Each arm 666a, 666b includes a distal segment 667a, 667b, wherein jaws 668a, 668b extend distally from distal segments 667a, 667b of arms 666a, 666b, respectively. Distal segments 667a, 667b of arms 666a, 666b define inwardly-facing or opposing cut-outs 669a, 669b, respectively. Each cut-out 669a, 669b is configured to receive a portion of pin 596 of inner drive sleeve 580 in the distal position of inner drive sleeve 580, as detailed below, thus enabling closer approximation of arms 666a, 666b and, thus, closer approximation of jaws 668a, 668b (see FIGS. 16-17). Further, cut-outs 669a, 669b may be defined by arcuate interior surface of distal segments 667a, 667b of arms 666a, 666b wherein the proximal portions of the arcuate surfaces defining cut-outs 669a, 669b are sloped complementary to the outer surfaces of pin 596 to facilitate pin 596 wedging arms 666a, 666b apart from one another upon proximal movement of inner drive sleeve 580 about end effector assembly 660.

With additional reference to FIGS. 16 and 17, in use, distal sliding of distal portion 584 of inner drive sleeve 580 about arms 666a, 666b of end effector assembly 660, e.g., in response to actuation of trigger 122 (FIG. 1), cams inner drive sleeve 580 about the exterior surfaces of arms 666a, 666b to urge arms 666a, 666b to pivot about pin 650 towards one another from the further-spaced position towards the closer-together position, thereby moving jaws 668a, 668b from the spaced-apart position towards the approximated position, as shown in FIG. 16, e.g., to form or close a surgical clip positioned therebetween about tissue disposed between jaws 668a, 668b. As jaws 668a, 668b approach the approximated position, pin 596 is received within cut-outs 669a, 669b to provide sufficient clearance between arms 666a, 666b and pin 596 to enable movement of jaws 668a, 558b to the approximated position.

Upon release or return of trigger 122 (FIG 1), as illustrated in FIG. 17, inner drive sleeve 580 is returned proximally and, thus, pin 596 is likewise returned proximally. As pin 596 is moved proximally between and relative to arms 666a, 666b, apex 597a of pin 596 eventually contacts the inwardly-facing surfaces of arms 666a, 666b and, as a result of the wedge-shaped configuration of pin 596 increasing in width in a proximal-to-distal direction, functions as a wedge against the inwardly-facing surfaces of arms 666a, 666b to urge arms 666a, 666b to pivot apart from one another, thus urging jaws 668a, 668b to pivot towards the spaced-apart position. The complementarily-sloped surfaces of pin 596 and cut-outs 669a, 669b, in embodiments further facilitate the wedging of arms 666a, 666b to return jaws 668a, 668b towards the spaced-apart position. Thus, pin 596 serves to return jaws 668a, 668b to the spaced-apart position upon release or return of trigger 122 (FIG. 1) to enable loading of a subsequent surgical clip for formation or closure about tissue. More specifically, pin 596 returns jaws 668a, 668b to the spaced-apart position without imparting a return biasing force that is required to be overcome in order to approximate jaws 668a, 668b. Thus, utilizing pin 596 to return jaws 668a, 668b to the spaced-apart position provides a decreased overall actuation force for approximating jaws 668a, 668b as a biasing spring is not required.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figures are presented only to demonstrate certain examples of the disclosure. Other elements, steps, methods and techniques that are insubstantially different from those described above and/or in the appended claims are also intended to be within the scope of the disclosure.

The invention may be described by reference to the following numbered paragraphs:-
1. A surgical clip applier, comprising:
   an outer shaft;
   an end effector assembly supported within the outer shaft and extending distally from the outer shaft, the end effector assembly including first and second jaw components coupled to one another at proximal portions of the first and second jaw components, the first and second jaw components including respective first and second arms extending distally from the respective proximal portions thereof and respective first and second jaws disposed at free ends of the first and second arms, respectively;
   an inner drive sleeve disposed within the outer shaft and about the first and second jaw components, the inner drive sleeve movable within the outer shaft and relative to the first and second jaw components from a proximal position to a distal position to cam about outer surfaces of the first and second jaw components to move the first and second jaws from a spaced-apart position to an approximated position to apply a surgical clip about tissue disposed between the first and second jaws; and
   a pin supported by the inner drive sleeve and disposed between the first and second arms of the first and second jaw components, wherein movement of the inner drive sleeve from the distal position back to the proximal position cams the pin about inner surfaces of the first and second jaw components to move the first and second jaws from the approximated position back to the spaced-apart position.
2. The surgical clip applier according to paragraph 1, wherein the pin defines a wedge-shaped configuration increasing in width in a proximal-to-distal direction to facilitate camming about the inner surfaces of the first and second jaw components to move the first and second jaws from the approximated position back to the spaced-apart position.
3. The surgical clip applier according to paragraph 2, wherein the pin defines a tear-drop-shaped configuration.
4. The surgical clip applier according to paragraph 1, wherein the arm of at least one of the first or second jaw components defines a cut-out within the inner surface thereof, the at least one cut-out configured to at least partially receive the pin therein in the approximated position of the first and second jaws.
5. The surgical clip applier according to paragraph 1, wherein the arm of each of the first and second jaw components defines a cut-out within the inner surface thereof, the cut-outs configured to at least partially receive the pin therein in the approximated position of the first and second jaws.
6. The surgical clip applier according to paragraph 1, wherein the first and second jaw components are pivotably coupled to one another and the outer shaft at the proximal portions thereof via a pivot pin, the first and second jaw components pivotable about the pivot pin to move the first and second jaws between the spaced-apart and approximated positions.
7. The surgical clip applier according to paragraph 6, wherein the pivot pin extends through opposed slots defined within the inner drive sleeve to enable slidable movement of the inner drive sleeve about the pivot pin.
8. A surgical clip applier, comprising:
   an outer shaft;
   an end effector assembly supported within the outer shaft and extending distally from the outer shaft, the end effector assembly including first and second jaw components movable between a spaced-apart position and an approximated position to apply a surgical clip about tissue disposed therebetween;
   an inner drive sleeve disposed within the outer shaft and about the first and second jaw components, the inner drive sleeve movable within the outer shaft and relative to the first and second jaw components from a proximal position to a distal position to cam about outer surfaces of the first and second jaw components to move the first and second jaw components from the spaced-apart position to the approximated position; and
   a wedge-shaped pin increasing in width in a proximal-to-distal direction supported by the inner drive sleeve and disposed between the first and second jaw components, wherein movement of the inner drive sleeve from the distal position back to the proximal position cams the pin about inner surfaces of the first and second jaw components to move the first and second jaw components from the approximated position back to the spaced-apart position.
9. The surgical clip applier according to paragraph 8, wherein the pin defines a tear-drop-shaped configuration.
10. The surgical clip applier according to paragraph 8, wherein at least one of the first or second jaw components defines a cut-out within the inner surface thereof, the at least one cut-out configured to at least partially receive the pin therein in the approximated position of the first and second jaw components.
11. The surgical clip applier according to paragraph 8, wherein each of the first and second jaw components defines a cut-out within the inner surface thereof, the cut-outs configured to at least partially receive the pin therein in the approximated position of the first and second jaw components.
12. The surgical clip applier according to paragraph 8, wherein the first and second jaw components are pivotably coupled to one another and the outer shaft via a pivot pin, the first and second jaw components pivotable about the pivot pin between the spaced-apart and approximated positions.
13. The surgical clip applier according to paragraph 12, wherein the pivot pin extends through opposed slots defined within the inner drive sleeve to enable slidable movement of the inner drive sleeve about the pivot pin.
14. A surgical clip applier, comprising:
   an outer shaft;
   an end effector assembly supported within the outer shaft and extending distally from the outer shaft, the end effector assembly including first and second jaw components movable between a spaced-apart position and an approximated position to apply a surgical clip about tissue disposed therebetween, at least one of the first or second jaw components defining a cut-out within an inner surface thereof; and
   an inner drive sleeve disposed within the outer shaft and about the first and second jaw components, the inner drive sleeve supporting a pin disposed between the first and second jaw components, the inner drive sleeve movable within the outer shaft and relative to the first and second jaw components from a proximal position to a distal position to cam about outer surfaces of the first and second jaw components to move the first and second jaw components from the spaced-apart position to the approximated position,
   wherein the pin is at least partially received within the at least one cut-out in the approximated position of the first and second jaw components, and wherein movement of the inner drive sleeve from the distal position back to the proximal position cams the pin about inner surfaces of the first and second jaw components to move the first and second jaw components from the approximated position back to the spaced-apart position.
15. The surgical clip applier according to paragraph 14, wherein the pin defines a wedge-shaped configuration increasing in width in a proximal-to-distal direction to facilitate camming about the inner surfaces of the first and second jaw components to move the first and second jaw components from the approximated position back to the spaced-apart position.
16. The surgical clip applier according to paragraph 15, wherein the pin defines a tear-drop-shaped configuration.
17. The surgical clip applier according to paragraph 15, wherein each of the first and second jaw components defines a cut-out within the inner surface thereof, the cut-outs configured to at least partially receive the pin therein in the approximated position of the first and second jaw components.
18. The surgical clip applier according to paragraph 14, wherein the first and second jaw components are pivotably coupled to one another and the outer shaft via a pivot pin, the first and second jaw components pivotable about the pivot pin between the spaced-apart and approximated positions.
19. The surgical clip applier according to paragraph 18, wherein the pivot pin extends through opposed slots defined within the inner drive sleeve to enable slidable movement of the inner drive sleeve about the pivot pin.

## Claims

1. A surgical clip applier, comprising:
an outer shaft;
an end effector assembly supported within the outer shaft and extending distally from the outer shaft, the end effector assembly including first and second jaw components coupled to one another at proximal portions of the first and second jaw components, the first and second jaw components including respective first and second arms extending distally from the respective proximal portions thereof and respective first and second jaws disposed at free ends of the first and second arms, respectively;
an inner drive sleeve disposed within the outer shaft and about the first and second jaw components, the inner drive sleeve movable within the outer shaft and relative to the first and second jaw components from a proximal position to a distal position to cam about outer surfaces of the first and second jaw components to move the first and second jaws from a spaced-apart position to an approximated position to apply a surgical clip about tissue disposed between the first and second jaws; and
a pin supported by the inner drive sleeve and disposed between the first and second arms of the first and second jaw components, wherein movement of the inner drive sleeve from the distal position back to the proximal position cams the pin about inner surfaces of the first and second jaw components to move the first and second jaws from the approximated position back to the spaced-apart position.

2. The surgical clip applier according to claim 1, wherein the pin defines a wedge-shaped configuration increasing in width in a proximal-to-distal direction to facilitate camming about the inner surfaces of the first and second jaw components to move the first and second jaws from the approximated position back to the spaced-apart position.

3. The surgical clip applier according to claim 2, wherein the pin defines a tear-drop-shaped configuration.

4. The surgical clip applier according to any preceding claim wherein the arm of at least one of the first or second jaw components defines a cut-out within the inner surface thereof, the at least one cut-out configured to at least partially receive the pin therein in the approximated position of the first and second jaws; preferably wherein the arm of each of the first and second jaw components defines a cut-out within the inner surface thereof, the cut-outs configured to at least partially receive the pin therein in the approximated position of the first and second jaws.

5. The surgical clip applier according to any preceding claim, wherein the first and second jaw components are pivotably coupled to one another and the outer shaft at the proximal portions thereof via a pivot pin, the first and second jaw components pivotable about the pivot pin to move the first and second jaws between the spaced-apart and approximated positions; preferably wherein the pivot pin extends through opposed slots defined within the inner drive sleeve to enable slidable movement of the inner drive sleeve about the pivot pin.

6. A surgical clip applier, comprising:
an outer shaft;
an end effector assembly supported within the outer shaft and extending distally from the outer shaft, the end effector assembly including first and second jaw components movable between a spaced-apart position and an approximated position to apply a surgical clip about tissue disposed therebetween;
an inner drive sleeve disposed within the outer shaft and about the first and second jaw components, the inner drive sleeve movable within the outer shaft and relative to the first and second jaw components from a proximal position to a distal position to cam about outer surfaces of the first and second jaw components to move the first and second jaw components from the spaced-apart position to the approximated position; and
a wedge-shaped pin increasing in width in a proximal-to-distal direction supported by the inner drive sleeve and disposed between the first and second jaw components, wherein movement of the inner drive sleeve from the distal position back to the proximal position cams the pin about inner surfaces of the first and second jaw components to move the first and second jaw components from the approximated position back to the spaced-apart position.

7. The surgical clip applier according to claim 6, wherein the pin defines a tear-drop-shaped configuration.

8. The surgical clip applier according to claim 7, wherein at least one of the first or second jaw components defines a cut-out within the inner surface thereof, the at least one cut-out configured to at least partially receive the pin therein in the approximated position of the first and second jaw components; preferably wherein each of the first and second jaw components defines a cut-out within the inner surface thereof, the cut-outs configured to at least partially receive the pin therein in the approximated position of the first and second jaw components.

9. The surgical clip applier according to claim 6, wherein the first and second jaw components are pivotably coupled to one another and the outer shaft via a pivot pin, the first and second jaw components pivotable about the pivot pin between the spaced-apart and approximated positions; preferably wherein the pivot pin extends through opposed slots defined within the inner drive sleeve to enable slidable movement of the inner drive sleeve about the pivot pin.

10. A surgical clip applier, comprising:
an outer shaft;
an end effector assembly supported within the outer shaft and extending distally from the outer shaft, the end effector assembly including first and second jaw components movable between a spaced-apart position and an approximated position to apply a surgical clip about tissue disposed therebetween, at least one of the first or second jaw components defining a cut-out within an inner surface thereof; and
an inner drive sleeve disposed within the outer shaft and about the first and second jaw components, the inner drive sleeve supporting a pin disposed between the first and second jaw components, the inner drive sleeve movable within the outer shaft and relative to the first and second jaw components from a proximal position to a distal position to cam about outer surfaces of the first and second jaw components to move the first and second jaw components from the spaced-apart position to the approximated position,
wherein the pin is at least partially received within the at least one cut-out in the approximated position of the first and second jaw components, and wherein movement of the inner drive sleeve from the distal position back to the proximal position cams the pin about inner surfaces of the first and second jaw components to move the first and second jaw components from the approximated position back to the spaced-apart position.

11. The surgical clip applier according to claim 10, wherein the pin defines a wedge-shaped configuration increasing in width in a proximal-to-distal direction to facilitate camming about the inner surfaces of the first and second jaw components to move the first and second jaw components from the approximated position back to the spaced-apart position.

12. The surgical clip applier according to claim 10, wherein the pin defines a tear-drop-shaped configuration.

13. The surgical clip applier according to claim 12, wherein each of the first and second jaw components defines a cut-out within the inner surface thereof, the cut-outs configured to at least partially receive the pin therein in the approximated position of the first and second jaw components.

14. The surgical clip applier according to claim 10, wherein the first and second jaw components are pivotably coupled to one another and the outer shaft via a pivot pin, the first and second jaw components pivotable about the pivot pin between the spaced-apart and approximated positions.

15. The surgical clip applier according to claim 14, wherein the pivot pin extends through opposed slots defined within the inner drive sleeve to enable slidable movement of the inner drive sleeve about the pivot pin.
